# NOUVEAU FASCICULE DE BREVET EUROPEEN

(11) **EP 0 665 006 B2**
(45) Date de publication et mention de la décision concernant l'opposition: **12.12.2007**
(45) Mention de la délivrance du brevet: 17.03.1999
(21) Numéro de dépôt: 94402597.2
(22) Date de dépôt: 16.11.1994
(51) Int. Cl.: A61K 8/18

(54) **Composition de teinture d'oxydation des fibres kératiniques comprenant un dérivé de paraphénylènediamine et un dérivé de méta-aminophenol, et procédé de teinture utilisant une telle composition**
Oxydative Haarfärbungszusammensetzung von einem Paraphenylendiamin und einem Meta-aminophenol, und Verfahren zur Anwendung
Oxydative hair dying composition comprising a paraphenylene diamine and a meta-aminophenol, and method of use

(30) Priorité: 24.01.1994 FR 9400702
(43) Date de publication de la demande: 02.08.1995
(73) Titulaire: L'ORÉAL, 75008 Paris (FR)
(72) Inventeur: Audousset, Marie-Pascale, F-92600 Asnieres (FR); Cotteret, Jean, F-78480 Verneuil S/Seine (FR)
(74) Mandataire: Bulle, Françoise

(56) Documents cités:
- EP-A- 0 007 537
- EP-A- 0 400 330
- FR-A- 2 315 255
- GB-A- 2 085 483
- US-A- 4 065 255
- US-A- 4 333 230
- US-A- 4 840 639

## Description

La présents invention concerne une composition de teinture d'oxydation des fibres kératiniques, et en particulier des fibres kératiniques humaines telles que les cheveux, comprenant en association, une paraphénylènediamine substituée en position 2 sur le noyau benzénique et un méta-aminophénol. Elle concerne également l'utilisation d'une telle composition.

Il est connu de teindre les fibres kératiniques et en particulier les cheveux humains, avec des compositions de teinture contenant des précurseurs de colorants d'oxydation, en particulier des ortho- ou paraphénylènediamines, des ortho-ou para-arninophénols, généralement appelés "bases d'oxydation", et des coupleurs encore appelés modificateurs de coloration, plus particulièrement des métaphénylènediamines, des méta-aminophénols et des métadiphénols, qui permettent de modifier et d'enrichir en reflets les colorations "de fond" obtenues par les produits de condensation des bases d'oxydation.

On recherche activement dans le domaine de la teinture capillaire d'oxydation, des précurseurs de colorants d'oxydation et des coupleurs capables d'engendrer, lorsqu'ils sont associés, une coloration rouge, ayant une résistance satisfaisante à la lumière, aux lavages, aux intempéries, à la transpiration et aux différents traitements que peuvent subir les cheveux.

Jusqu'ici ces colorations ont été obtenues avec des teintures à base de paraphénylènediamine. Mais actuellement, l'utilisation de la paraphényiène-diamine est remise en question pour des raisons toxicologiques.

Il a également été proposé, notamment dans les demandes de brevet FR-A-2 315 255 et EP-A-0 007 537, des compositions pour la teinture d'oxydation des fibres kératiniques contenant l'association de certaines paraphénylènediamines à titre de base d'oxydation et de certains métaaminophénols à titre de coupleurs, cependant de telles compositions ne sont pas entièrement satisfaisantes, notamment en ce qui concerne la résistance des colorations obtenues vis à vis des différentes agressions que peuvent subir les fibres kératiniques.

Or, après d'importantes recherches menées sur la question, la Demanderesse vient maintenant de découvrir qu'il est possible d'obtenir de nouvelles teintures non toxiques, qui engendrent des colorations allant du rouge au pourpre, à la fois intenses et résistantes, en associant une paraphénylènediamine substituée en position 2 sur le noyau benzénique et un méta-aminophénol, tels que définis ci-après.

La présente invention a ainsi pour objet une composition de teinture pour fibres kératiniques, en particulier pour fibres kératiniques humaines telles que les cheveux, du type comprenant, dans un milieu approprié pour la teinture, un seul précurseur de colorant d'oxydation et au moins un coupleur, et caractérisée par le fait qu'elle contient, à titre de précurseur de colorant d'oxydation, la 2-(β-hydroxyethyl) paraphénylènediamine ou l'un de ses d'addition avec un acide et le 2-méthyl 5-N-(β-hydroxyéthyléthylamino)phénol ou l'un de ses sels d'addition avec un acide à titre de coupleur.

Les nouvelles teintures ainsi obtenues permettent d'aboutir à des colorations allant du rouge au pourpre, soutenues, non toxiques, et résistant à la fois à la lumière, aux lavages, aux intempéries, à la transpiration et aux différents traitements que peuvent subir les cheveux. Elles sont tout particulièrement très résistantes aux shampooings.

Un autre objet de l'invention porte sur la composition prête à l'emploi, contenant les différents agents utilisés pour la teinture des libres kératiniques définis ci-dessus et un agent oxydant.

L'invention vise également un procédé de teinture des fibres kératiniques, et en particulier des fibres kératiniques humaines telles que les cheveux, consistant à appliquer sur ces fibres au moins une composition (A) contenant, dans un milieu approprié pour la teinture, un seul précurseur de colorant d'oxydation et un coupleur tels qu'ils ont été définis ci-avant, la couleur étant révélée à pH alcalin neutre ou acide à l'aide d'un agent oxydant qui est ajouté juste au moment de l'emploi à la composition (A) ou qui est présent dans une composition (B) appliquée simultanément ou séquentiellement de façon séparée.

L'invention a également pour objet des dispositifs de teinture ou "kits" à plusieurs compartiments, dont le premier compartiment contient la paraphénylènediamine définie ci-dessus, à titre de seul précurseur de colorant d'oxydation et au moins le coupleur defini ci-dessus, et le deuxième compartiment, un agent oxydant.

D'autres caractéristiques, aspects, objets et avantages de l'invention apparaîtront plus clairement à la lecture de la description et des exemples qui suivent.

Les sels d'acide qui peuvent être utilisés selon l'invention sont choisis de préférence parmi les chlorhydrates, les sulfates, les bromhydrates et les tartrates.

La concentration en précurseur ou ses sels peut varier entre 0.01 et 10 % en poids environ par rapport au poids total de la composition de teinture, et de préférence entre 0,05 et 5 % en poids environ.

La concentration en coupleur ou ses sels tels que définis ci-dessus peut varier entre 0,005 et 3% en poids environ par rapport au poids total de la composition de teinture, et de préférence entre 0,05 et 2 % en poids environ.

L'agent oxydant est choisi de préférence parmi le peroxyde d'hydrogène, le peroxyde d'urée, les bromates de métaux alcalins, les persels tels que les perborates et les persulfates. L'utilisation du peroxyde d'hydrogène est particulièrement préférée.

La composition (A), qui renferme l'association des colorants telle que décrite ci-dessus, peut avoir un pH compris entre 3 et 11, qui peut être ajusté à la valeur choisie au moyen d'agents alcalinisants habituellement utilisés en teinture des fibres kératiniques, tels que l'ammoniaque, les carbonates alcalins, les alcanolamines telles que par exemple les mono-, di- et triéthanolamines ainsi que leurs dérivés, les hydroxydes de sodium ou de potassium, les composés de formule : 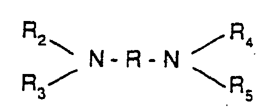 dans laquelle R est un reste propylène éventuellement substitué par un groupement hydroxyle ou un radical alkyle en C₁-C₄ ; R₂ R₃, R₄ et R₅, simultanément ou indépendamment l'un de l'autre, représentent un atome d'hydrogène, un radical alkyle en C₁-C₄ ou hydroxyalkyle en C₁-C₄,
soit au moyen d'agents acidifiants classiques, tels que les acides minéraux ou organiques comme par exemple les acides chlorhydrique, tartrique, citrique et phosphorique.

Le pH de la composition (B) renfermant l'agent oxydant tel que défini ci-dessus, est tel qu'après un mélange avec la composition (A), le pH de la composition appliquée sur les fibres kératiniques humaines varie de préférence entre 3 et 11. Il est ajusté à la valeur désirée à l'aide d'agents acidifiants ou éventuellement alcalinisants bien connus de l'état de la technique, tels que ceux décrits ci-dessus.

La composition oxydante (B) est de préférence constituée par une solution d'eau oxygénée.

Selon un mode de réalisation préféré du procédé de teinture de l'invention, on mélange, au moment de l'emploi, la composition de teinture (A) décrite ci-dessus avec une solution oxydante en une quantité suffisante pour développer une coloration. Le mélange obtenu est ensuite appliqué sur les fibres kératiniques humaines et on laisse poser pendant 5 à 40 minutes, de préférence 15 à 30 minutes, après quoi on rince, on lave au shampooing, on rince à nouveau et on sèche.

Les compositions de teinture peuvent encore contenir, en plus des colorants définis ci-dessus, d'autres coupleurs et/ou des colorants directs, notamment pour modifier les nuances ou les enrichir en reflets.

Les compositions de teinture peuvent également contenir des agents antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la tertiobutyl hydroquinone et l'acide homogentisique et sont alors généralement présents dans des proportions comprises entre environ 0,05 et 1,5 % en poids par rapport au poids total de la composition.

Les compositions de teinture contiennent également, dans leur forme de réalisation préférée, des agents tensioactifs bien connus de la technique, dans des proportions comprises entre environ 0,5 et 55 % en poids, et de préférence entre 2 et 50 % en poids, par rapport au poids total de la composition, des solvants organiques, dans des proportions comprises entre environ 1 et 40 % en poids, et en particulier entre 5 et 30 % en poids, par rapport au poids total de la composition, ou tout autre adjuvant cosmétiquement acceptable et connu de la technique antérieure en teinture d'oxydation capillaire.

La composition appliquée sur les cheveux peut se présenter sous des formes diverses, telles que sous forme de liquide, de crème, de gel, ou sous toute autre forme appropriée pour réaliser une teinture des fibres kératiniques, et notamment des cheveux humains. En particulier, elle peut être conditionnée sous pression en flacon aérosol en présence d'un agent propulseur et former une mousse.

Des exemples concrets illustrant l'invention vont maintenant être donnés. On commencera par définir les tests utilisés pour évaluer les performances des teintures d'oxydation selon l'invention, concernant leur résistance à la transpiration, à la lumière et aux shampooings, aux intempéries ou à la permanente.

### Résistance à la transpiration :

On utilise une solution de sueur synthétique de composition suivante : 10 g de NaCl, 1 g d'hydrogénophosphate de potassium, 0,25 g d'histidine, de l'acide lactique jusqu'à pH = 3,2 et de l'eau distillée pour compléter à 100 g.

Dans un cristallisoir recouvert d'un verre de montre et renfermant cette solution de sueur, on immerge les mèches de cheveux teints qu'on laisse séjourner de 20 à 50 heures à 37° C. On rince ensuite les mèches et on les sèche.

### Résistance à la lumière (Xenotest) :

Les cheveux teints sont fixés sur un support (carton ou plastique). Ces supports sont disposés sur des porte-échantillons qui tournent autour d'une lampe Xénon pendant une durée variant de 20 à 80 heures sous un taux d'humidité variant de 25 à 75% HR (Humidité Relative) et à une température de 25° C.

### Résistance aux shampooings (machine Ahiba-Texomat) :

On place des mèches de cheveux teints dans un panier que l'on immerge dans une solution d'un shampooing standard. Le panier est soumis à un mouvement de va-et-vient vertical de fréquence variable ainsi qu'à un mouvement de rotation qui reproduisent l'action d'un frottement manuel, ce qui engendre la formation de mousse.

Après 3 minutes d'épreuve, on retire les mèches que l'on rince puis sèche. Les mèches teintes peuvent être soumises à plusieurs épreuves shampooings consécutives.

### Résistance aux intempéries (Epreuve combinée):

Les mèches teintes sont exposées à la lumière forte ( Xenotest 40 h ), sous une humidité relative de 60 %, et simultanément, toutes les 12 heures, et pendant une durée de 20 minutes, elles sont soumises à une aspersion d'eau.

### Résistance à la permanente :

Les mèches teintes sont immergées dans une solution réductrice de permanente Dulcia Vital (L'Oréal), de force variant de 1 à 3, pendant une durée variant de 10 à 20 minutes; les mèches sont rincées; on les trempe ensuite dans une solution de fixateur (oxydant), pendant 5 minutes. Après rinçage à l'eau, lavage au shampooing standard et rinçage à l'eau, on les sèche.

### EXEMPLE :

On a préparé la composition de teinture, conforme à l'invention, suivante :

| | |
|---|---|
| - 2-(β-hydroxyéthyl) paraphénylènediamine, dichlorhydrate | 0,675 g |
| - 2-méthyl 5-N-(β-hydroxyéthylamino) phénol | 0,501 g |
| - Alcool oléique polyglycérolé à 2 moles de glycérol | 4,0 g |
| - Alcool oléique polyglycérolé à 4 moles de glycérol (78 % de MA) | 5,7 g MA |
| - Acide oléique | 3,0 g |
| - Amine oléique à 2 moles d'oxyde d'éthylène, vendue sous la dénomination Ethomeen 012 par la Société AKZO | 7,0 g |
| - Laurylaminosuccinamate de diéthylaminopropyle, sel de sodium à 55% de MA | 3,0 g MA |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse à 35% de MA | 0,4 g MA |
| - Acétate d'ammonium | 0,8 g |
| - Antioxydant, séquestrant, | qs |
| - Parfum, conservateur, | qs |
| - Ammoniaque à 20% de NH₃ | 10,0 g |
| - Eau déminéralisée qsp | 100 g |

Au moment de l'emploi, on a mélangé cette composition poids pour poids avec de l'eau oxygénée titrant 20 volumes (6% en poids), de pH 3.

On a obtenu un mélange de pH 9,8.

On a ensuite appliqué ce mélange sur des cheveux gris à 90% de blancs, pendant 30 minutes. Après rinçage, lavage au shampooing, rinçage et séchage, les cheveux sont teints dans une nuance rouge-pourpre qui résiste en particulier remarquablement bien aux shampooings.

## Revendications

1. Composition de teinture d'oxydation pour fibres kératiniques, en particulier pour fibres kératiniques humaines telles que les cheveux, du type comprenant, dans un milieu approprié pour la teinture, un seul précurseur de colorant d'oxydation et au moins un coupleur, **caractérisée par le fait qu'**elle contient, à titre de précurseur de colorant d'oxydation la 2-(β-hydroxyéthyl) paraphénylènediamine ou l'un de ses sels d'addition avec un acide et le 2-méthyl 5-N-(β-hydroxyéthylamino) phénol à titre de coupleur ou l'un de ses sels d'addition avec un acide.

2. Composition de teinture selon la revendication 1, **caractérisée par le fait que** les sels d'addition avec un acide sont choisis parmi les chlorhydrates, les sulfates, les bromhydrates et les tartrates.

3. Composition de teinture selon l'une des revendications 1 ou 2, **caractérisée par le fait que** la paraphénylènediamine ou ses sels est présente dans une concentration comprise entre 0,01 et 10 % en poids environ par rapport au poids total de la composition, de préférence entre 0,05 et 5 % en poids environ, et que le coupleur ou ses sels, est présent dans une concentration comprise entre 0,005 et 3 % en poids environ, de préférence entre 0,05 et 2 % en poids environ, par rapport au poids total de la composition.

4. Composition de teinture selon l'une quelconque des revendications 1 à 3, prête à l'emploi, **caractérisée en ce qu'**elle contient en outre un agent oxydant et qu'elle possède un pH compris entre 3 et 11.

5. Procédé de teinture des fibres kératiniques et en particulier des fibres kératiniques humaines telles que les cheveux, **caractérisé par le fait qu'**il consiste à appliquer sur les fibres une composition de teinture (A) selon l'une quelconque des revendications 1 à 4, et à révéler la couleur en milieu alcalin neutre ou acide à l'aide d'un agent oxydant qui est ajouté juste au moment de l'emploi à cette composition(A) ou qui est présent dans une composition (B) appliquée simultanément ou séquentiellement de façon séparée.

6. Dispositif à plusieurs compartiments ou "Kit" pour la teinture des fibres kératiniques et en particulier des fibres kératiniques humaines telles que les cheveux, **caractérisé par le fait qu'**il comporte au moins deux compartiments, dont l'un d'entre eux renferme une composition (A) telle que définie dans l'une quelconque des revendications 1 à 3 et un autre une composition (B) comprenant un agent oxydant dans un milieu approprié pour la teinture.

7. Utilisation d'une composition de teinture selon l'une quelconque des revendications 1 à 4 ou d'un dispositif de teinture ou "Kit" à plusieurs compartiments selon la revendication 6, pour la teinture des fibres kératiniques humaines telles que les cheveux.

## Claims

1. Oxidation dye composition for keratinous fibres, in particular for human keratinous fibres such as hair, of the type comprising, in a medium which is suitable for dyeing, only one oxidation dye precursor and at least one coupling agent, and which composition is **characterized in that** it contains, as oxidation dye precursor, 2-(β-hydroxyethyl)-paraphenylenediamine or one of the addition salts thereof with an acid and, as coupling agent, 2-methyl-5-N-(β-hydroxyethylamino)phenol or one of the addition salts thereof with an acid.

2. Dye composition according to claim 1, **characterized in that** the addition salts with an acid are chosen from hydrochlorides, sulphates, hydro-bromides and tartrates.

3. Dye composition according to one of claims 1 or 2, **characterized in that** the paraphenylenediamine, or the salts thereof, is present in a concentration of between 0.01 and 10 % by weight approximately relative to the total weight of the composition, preferably between 0.05 and 5 % by weight approximately, and that the coupling agent, or the salts thereof, is present in a concentration of between 0.005 and 3 % by weight approximately, preferably between 0.05 and 2 % by weight approximately, relative to the total weight of the composition.

4. Dye composition according to any one of claims 1 to 3, which is a ready-to-use composition, **characterized in that** it additionally contains an oxidizing agent and **in that** it has a pH between 3 and 11.

5. Process for dyeing keratinous fibres and in particular human keratinous fibres such as hair, **characterized in that** it consists in applying to the fibres a dye composition (A) according to any one of claims 1 to 4, and in developing the colour in an alkaline, neutral or acidic medium using an oxidizing agent which is added only at the time of use to this composition (A) or which is present in a composition (B) that is applied simultaneously or sequentially in a separate manner.

6. Device containing several compartments, or "kit", for dyeing keratinous fibres and in particular human keratinous fibres such as hair, **characterized in that** it is composed of at least two compartments, one of which contains a composition (A) as defined in any one of claims 1 to 3, and another of which contains a composition (B) comprising an oxidizing agent in a medium which is suitable for dyeing.

7. Use of a dye composition according to any one of claims 1 to 4 or of a dyeing device, or "kit", containing several compartments according to claim 6, for dyeing human keratinous fibres such as hair.

## Patentansprüche

1. Zusammensetzung zum oxidativen Färben von Keratinfasern und insbesondere menschlichen Keratinfasern, wie dem Haar, des Typs, der in einem zum Färben geeigneten Medium einen einzigen Precursor eines Oxidationsfarbstoffs und mindestens einen Kuppler enthält, **dadurch gekennzeichnet, daß** sie als Precursor des Oxidationsfarbstoffs 2-(β-Hydroxyethyl)-p-phenylendiamin oder eines seiner Additionssalze mit einer Säure und als Kuppler 2-Methyl-5-N-(β-hydroxyethylamino)-phenol oder eines seiner Additionssalze mit einer Säure enthält.

2. Zusammensetzung zum Färben nach Anspruch 1, **dadurch gekennzeichnet, daß** die Additionssalze mit einer Saüre unter Hydrochloriden, Sulfaten, Hydrobromiden und Tartraten ausgewählt sind.

3. Zusammensetzung zum Färben nach einem der Ansprüche 1 oder 2, **dadurch gekennzeichnet, daß** das p-Phenylendiamin oder seine Salze in einer Konzentration im Bereich von etwa 0,01 bis 10 Gew.-%, bezogen auf das Gesamtgewicht der Zusammensetzung, und vorzugsweise im Bereich von etwa 0,05 bis 5 Gew.-% vorliegen und daß der Kuppler oder seine Salze in einer Konzentration im Bereich von etwa 0,005 bis 3 Gew.-%, vorzugsweise im Bereich von etwa 0,05 bis 2 Gew.-%, bezogen auf das Gesamtgewicht der Zusammensetzung, vorliegen.

4. Gebrauchsfertige Zusammensetzung zum Färben nach einem der Ansprüche 1 bis 3, **dadurch gekennzeichnet, daß** sie ferner ein Oxidationsmittel enthält und daß sie einen pH-Wert im Bereich von 3 bis 11 aufweist.

5. Verfahren zum Färben von Keratinfasern und insbesondere menschlichen Keratinfasern, wie dem Haar, **dadurch gekennzeichnet, daß** es darin besteht, auf die Fasern eine Zusammensetzung zum Färben (A) nach einem der Ansprüche 1 bis 4 aufzubringen und in einem alkalischen, neutralen oder sauren Medium mit einem Oxidationsmittel, das unmittelbar bei der Anwendung zu dieser Zusammensetzung (A) gegeben wird oder das in einer Zusammensetzung (B) vorliegt, die gleichzeitig oder nachfolgend getrennt davon aufgebracht wird, die Farbe zu entwickeln.

6. Vorrichtung zum Färben von Keratinfasern und insbesondere menschlichen Keratinfasern, wie dem Haar, oder 'Kit' mit mehreren Abteilungen, darduch **gekennzeichnet**, daß sie mindestens zwei Abteilungen aufweist, wobei eine der Abteilungen eine in einem der Ansprüche 1 bis 3 definierte Zusammensetzung (A) und die andere Abteilung eine Zusammenzetzung (B) enthält, die in einem zum Färben geeigneten Medium ein Oxidationsmittel enthält.

7. Verwendung einer Zusammensetzung zum Färben nach einem der Ansprüche 1 bis 4 oder einer Vorrichtung zum Färben oder eines 'Kits' mit mehreren Abteilungen nach Anspruch 6 zum Färben von menschlichen Keratinfasern, wie dem Haar.
